# EUROPEAN PATENT SPECIFICATION

(11) **EP 4 175 935 B1**
(45) Date of publication and mention of the grant of the patent: **04.03.2026**
(21) Application number: 20736682.4
(22) Date of filing: 03.07.2020
(51) Int. Cl.: C07C 17/10, C07C 17/12, C07C 17/25, C07C 17/383, C07C 21/06, C07C 19/045, B01J 23/745, B01J 35/61, B01J 35/63, B01J 35/64

(54) **IMPROVED PROCESS OF REMOVING BENZENE AND CHLOROPRENE FROM A VINYL CHLORIDE COMPRISING PROCESS STREAM**
VERFAHREN ZUR ENTFERNUNG VON BENZOL UND CHLOROPREN AUS EINEM VINYLCHLORID ENTHALTENDEN PROZESSSTROM
PROCÉDÉ AMÉLIORÉ D'ÉLIMINATION DU BENZÈNE ET DU CHLOROPRÈNE DANS UN COURANT DE TRAITEMENT COMPRENANT DU CHLORURE DE VINYLE

(43) Date of publication of application: 10.05.2023
(73) Proprietor: Vestolit GmbH, 45772 Marl (DE)
(72) Inventor: SCHILLGALIES, Ingo, 48308 Senden (DE); NEU, Thomas, 48249 Dülmen (DE); BAHLMANN, Antje, 46325 Borken (DE); HELLMANN, Eberhard, 45964 Gladbeck (DE); JANSEN, Alexander, 46282 Dorsten (DE); KLEINE, Katharina, 48249 Dülmen (DE); ECKELT, Reinhard, 18059 Rostock (DE); KOECKRITZ, Angela, 12437 Berlin (DE)
(74) Representative: Godemeyer Blum Lenze Patentanwälte Partnerschaft mbB - werkpatent
(86) International application number: PCT/EP2020/068801
(87) International publication number: WO 2022/002412

(56) References cited:
- DE-A1- 4 129 391
- DE-A1- 4 139 632
- US-A- 5 068 475

## Description

The invention relates to a process of removing by-products from a vinyl chloride comprising process stream from a 1,2-dichloroethane thermal cracking process. The invention also relates to the use of a specific heterogeneous iron-aluminium(oxide)-catalyst in a process of removing by-products from such process stream following a 1,2-dichloroethane thermal cracking process.

Vinyl chloride is a valuable commercial product used foremost for the production of polyvinyl chloride, one of the most important synthetic polymer plastics. Vinyl chloride is, therefore, also referred to as vinyl chloride monomer (VCM). One process for producing VCM involves the thermal cracking (thermal dehydrochlorination or "cracking") of 1,2-dichloroethane (EDC), wherein EDC splits, at temperatures of about 450 to 600 °C, into VCM and hydrogen chloride. The obtained VCM and the hydrogen chloride are subsequently separated in respective distillation units, after which the hydrogen chloride is recycled, for instance for EDC production. The cracking of EDC is incomplete and yields a process stream comprising a mixture of the main products mentioned above, along with uncracked EDC and by-products of various chemicals. The uncracked EDC can be recycled and send back for thermal cracking. However, the by-products, if circulated back to the thermal cracking process, are known to have severe adverse effects on the reaction kinetics, such as to increase coke formation and, consequently, to increase maintenance costs. This leads to the problem of effectively separating the uncracked EDC from the by-products.

The by-products comprise low-boiling compounds, such as chloroprene and benzene, and high-boiling compounds. Some of these low-boiling compounds can be removed by distillation, whilst others, especially benzene, have very close boiling temperatures to EDC; to avoid their difficult and energy-consuming separation, these low-boiling compounds have to be converted to high-boiling compounds, which then can easily be removed through distillation.

Generally, these low-boiling compounds can be converted to high-boiling compounds by chlorination. In this respect, low-boiling compounds refer to compounds having a boiling temperature of below the boiling temperature of EDC, which is around 84 °C (at 1.01325 bar), while high-boiling compounds refer to compounds having a boiling temperature of above the boiling temperature of EDC. Chloroprene can be converted to high-boiling chlorobutane by thermal chlorination, whilst benzene requires a catalyst for its chlorination. However, excess chlorination of benzene needs to be avoided to reduce the cost of the chlorination process, but also because the presence of hexachlorbenzene interferes with the subsequent distillation step due to its sublimation. Homogenous catalysts, such as iron(III) chloride, which can be produced in situ by chlorination of iron fillers, are usually employed since the cracking of EDC produces coke that can cause a blocking and deactivation of heterogeneous catalysts. When using iron(III) chloride, only a partial flow of the process stream can be chlorinated, as otherwise too much iron would be introduced into the process. Hence, following the thermal chlorination, whereby chloroprene is converted to chlorobutane, a partial flow of the product mixture comprising uncracked EDC, benzene and remaining unconverted chloroprene is catalytically chlorinated using iron(III) chloride, whereby benzene is converted to chlorobenzene. These high-boiling compounds are separated from the uncracked EDC, which then can be used for further thermal cracking.

It has been found that the use of homogenous iron catalysts, in particular of iron(III) chloride, causes serious problems downstream of the chlorination. In an undesired reaction, iron(III) chloride leads to chlorination of EDC to trichlorethene (TCE). Further, iron catalyses the cracking of EDC to coke, leading to blocking of reactors and columns in subsequent process steps. Iron is also flushed out and accumulates downstream, additionally leading to the blocking of reactors and columns.

In US 5,068,475 A, a process is described for chlorinating benzene in the presence of an iron catalyst, being finely divided iron turnings, iron baskets or steel wool, that fills the entire reaction space and produces minimal amount of active iron(III) chlorine.

In DE 40 33 047 A1, a process is described wherein, following the separation of VCM and hydrogen chloride, the process stream is first divided into a process stream (I), containing no high-boiling compounds, and a process stream (II), containing high-boiling compounds. The process stream (I) is treated with chorine in the presence of metallic iron. Alternatively, the process stream (II) can also be chlorinated. Both process streams are then reunited and EDC is recovered. In DE 41 39 632 A1, the process described in DE 40 33 047 A1 is modified by treating the reunited process streams once more with chlorine before EDC is recovered. In DE 41 29 391 A1, the process described in DE 40 33 047 A1 is further modified as it has been found that the used iron catalyst still leads to severe downstream problems by causing for instance deposits on heat exchangers. To circumvent the use of metallic iron, a γ-aluminium(oxide) catalyst, preferably as moulding such as spheres, cylinders or rings, is employed for chlorinating the process stream (I).

The prior art processes known to us have, however, the disadvantage that the catalytical chlorination is not selective enough, producing unwanted chlorinated products such as hexachlorbenzene and TCE.

Therefore, it is an object of this invention to provide a new or improved process of removing by-products from a vinyl chloride comprising process stream from a 1,2-dichloroethane thermal cracking process.

This object is solved by the present invention, namely a process of removing by-products from a vinyl chloride comprising process stream from a 1,2-dichloroethane thermal cracking process comprising the steps of:
(a) providing a starting material comprising 1,2-dichloroethane;
(b) thermally cracking 1,2-dichloroethane by heating the starting material to obtain a product mixture comprising vinyl chloride, hydrogen chloride, uncracked 1,2-dichloroethane and by-products;
(c) separating the hydrogen chloride and separating the vinyl chloride from the product mixture thereby obtaining a process stream comprising uncracked 1,2-dichloroethane and by-products;
(d) chlorinating the obtained process stream by adding liquid and/or gaseous chlorine to the process stream in the presence of a heterogeneous catalyst, thereby obtaining a process stream comprising the uncracked 1,2-dichloroethane, chlorinated by-products and, optionally, still by-products from step c);
(e) recovering the uncracked 1,2-dichloroethane from the process stream by separating the chlorinated by-products;
(f) optionally regenerating the catalyst through backflushing; and
wherein the heterogeneous catalyst is an iron-aluminium(oxide)-catalyst with a formula FeₐAl_{b}O_{c}.

It has been found that using a heterogeneous iron-aluminium(oxide)-catalyst with a formula FeₐAl_{b}O_{c} has the advantage of converting chloroprene and benzene in a single process step. This allows for a considerable reduction of plant size and complexity. Further, chloroprene and benzene are converted in a highly selective reaction, hence benzene is not or scarcely converted to unwanted hexachlorbenzene. Even more, the loss of uncracked EDC through unwanted chlorination is minimized, and, as a consequence, less coke is formed. Another advantage of the heterogeneous iron-aluminium(oxide)-catalyst is that it is not flushed out compared to a homogeneous catalyst; and, thus, less iron accumulates in the downstream processes, leading to an increase runtime of the downstream reactors and columns and reduced maintenance costs.

The iron-aluminium(oxide)-catalyst with the formula FeₐAl_{b}O_{c} has preferably the formula Fe₍₁₋₅₎Al₍₁₋₂₅₎O₍₁₋₃₀₎.

The starting material EDC of step (a) is optionally provided by direct chlorination and/or oxychorination, both reactions being exothermic. Direct chlorination is conducted at a temperature of, for example, 40 to 130 °C at 2.6 bar, preferably 100 °C at 2.6 bar, wherein chlorine and ethylene are catalytically converted:

C₂H₄ + Cl₂ → C₂H₄Cl₂

Direct chlorination has the advantage of producing, in a single reactor, EDC that can be used directly for step (b) thermally cracking the EDC, without any further purification necessary. Alternatively, oxychorination can be used to produce EDC in a reaction, at a temperature of, for example, above 200 °C at 2.8 bar, preferably 230 °C at 2.8 bar, wherein ethylene, hydrogen chloride and oxygen are catalytically converted:

C₂H₄ + 2HCI + ½ O₂ → C₂H₄Cl₂ + H₂O

EDC produced in this way must be purified, for instance by distillation, before being used for step (b) of the process. EDC can be providing by direct chlorination, oxychlorination, or a combination thereof, or in any other suitable way. Preferably, the starting material in step (a) comprises 70 wt.% or more, 80 wt.% or more or 90 wt.% or more EDC. Further preferably, the starting material consists of EDC.

In the next process step (b), EDC is thermally cracked, in an endothermic reaction, by a temperature ranging from, for example, 450 to 650 °C, preferably 450 to 600 °C, further preferably 450 to 550 °C, preferably 480 °C. Thereby a product mixture is obtained comprising vinyl chloride and hydrogen chloride:

C₂H₄Cl₂ → C₂H₃Cl + HCl

The cracking reaction in step (b) is incomplete, having a conversion rate of, for example, 20 to 99 wt.%, preferably 40 to 70 wt.%, further preferably 50 to 60 wt.% based on the total throughput of EDC. The product mixture obtained in step (b) further comprises uncracked 1,2-dichloroethane and by-products.

The by-products of the obtained product mixture of step (b) preferably comprise one or more hydrocarbons selected from the list comprising benzene, chloroprene and other hydrocarbons.

In step (c), the vinyl chloride and the hydrogen chloride are separated from the product mixture, preferably through distillation. Thereafter, hydrogen chloride may be recycled and, for instance, fed to the oxychlorination reaction for EDC production, while the vinyl chloride is optionally stored.

Following the removal of vinyl chloride and hydrogen chloride, a process stream is obtained that comprises the uncracked 1,2-dichloroethane and the by-products, wherein the by-products preferably comprise one or more hydrocarbons selected from the list comprising benzene, chloroprene and other hydrocarbons.

In step (d), this process stream is chlorinated by adding liquid and/or gaseous chlorine in the presence of a heterogeneous catalyst being an iron-aluminium(oxide)-catalyst with a formula FeₐAl_{b}O_{c}. Thereby, a process stream comprising the uncracked 1,2-dichloroethane, chlorinated by-products and, optionally, still by-products from step (c) is obtained.

In an embodiment, in step (d) the chlorinated by-products comprise high-boiling chlorinated by-product, having a boiling temperature of at least 85°C, such as high-boiling chlorobenzene and high-boiling chlorobutane.

Surprisingly, by employing the iron-aluminium(oxide)-catalyst with the formula FeₐAl_{b}O_{c}, it is possible to chlorinate by-products in a single step, without the need to divide the process stream in a partial stream. The required plant size and complexity is therefore reduced. Moreover, this chlorination step is shown to be highly selective, resulting in chlorinated by-products, such that EDC is not further chlorinated in an undesired side reaction and such that no hexachlorbenzene is generated. Preferably, EDC is not converted to 1,1,2-trichloroethene. Preferably, benzene is not converted to hexachlorbenzene. In consequence, less coke is produced and runtime between decoking and maintenance intervals can be increased.

Furthermore, the iron-aluminium(oxide)-catalyst with a formula FeₐAl_{b}O_{c} is preferably not flushed out and does not accumulate in downstream reactors or columns, advantageously leading to even longer runtimes and lower maintenance costs.

The iron-aluminium(oxide)-catalyst can be used preferably in a catalytic fixed bed reactor, but may also be used in other suitable reactors.

A suitable temperature for carrying out the chlorination in step (d) is, for example, 20 to 100 °C, preferably 40 to 90 °C, further preferably 60 to 80 °C. A suitable pressure is, for example, 0.5 to 3 bar, preferably 0.5 to 2 bar.

A suitable amount of chlorine per kg EDC for carrying out the chlorination of step (d) is preferably 0.1 to 20 g, preferably 1.5 to 15 g, further preferably 2 to 4 g. In a preferred embodiment, in step (d) the chlorine is provided as a gaseous chlorine.

The benzene conversion rate to high-boiling chlorobutane is preferably 99%, further preferably 100 %.

Following the catalytic chlorination in step (d), the process stream contains below 2000 ppm, preferably below 1000 ppm, further preferably 500 ppm, low-boiling benzene and/or low-boiling chloroprene.

In the subsequent process step (e), the uncracked EDC is recovered by separating the chlorinated by-products.

In a preferred embodiment, in step (e) the uncracked EDC is recovered by separating the chlorinated by-products through distillation. Preferably, the chlorinated by-products comprise high-boiling chlorinated by-products, having a boiling temperature of at least 85°C.

In process step (f), the heterogeneous iron-aluminium(oxide)-catalyst with a formula FeₐAl_{b}O_{c} can optionally be regenerated through backflushing. This backflushing can be conducted by flushing the catalyst using a gaseous composition and/or a liquid, which do not react with the catalyst surface. In a preferred embodiment, the catalyst is backflushed one or more times with a flush-liquid/flush gas. The flush-liquid/flush gas can be a process stream and/or a liquid/gas comprising preferably chlorinated or non-chlorinated saturated hydrocarbons and/or chlorinated or non-chlorinated unsaturated hydrocarbons with/without additives. The backflushing is preferably conducted at a temperature of 20 to 150 °C, preferably 30 to 100 °C.

It is another object of the invention to provide the use of a heterogeneous iron-aluminium(oxide)-catalyst in the process described above. In particular, the use of a heterogeneous iron-aluminium(oxide)-catalyst for removing by-products from a vinyl chloride comprising process stream from a 1,2-dichloroethane thermal cracking process characterized in that the catalyst is an iron-aluminium(oxide)-catalyst with a formula FeₐAl_{b}O_{c}, preferably with the formula Fe₍₁₋₅₎Al₍₁₋₂₅₎O₍₁₋₃₀₎.

Through this use the by-products are preferably chlorinated by adding liquid and/or gaseous chlorine to the process stream in the presence of the iron-aluminium(oxide)-catalyst according to the process and its preferred embodiments as described above.

### Examples

### Example 1: Chlorination using iron(III) chloride as catalyst

The catalytic tests were carried out according to the method defined under experimental procedures. The results of the screening are listed in table 1.

**Table 1: Chlorination using iron(III) chloride as catalyst**

| Catalyst [mol%] | T [°C] | Time [h] | 2-CP peak area [%] | EDC peak area [%] | BE peak area [%] | TCE peak area [%] | CB peak area [%] | HCB peak area [%] |
|---|---|---|---|---|---|---|---|---|
| Crude EDC | | | 0.3 | 98.08 | 1.05 | 0.04 | 0.07 | 0.00 |
| blank | 60 | 3 | 0.0 | 84.98 | 0.49 | 12.79 | 0.18 | 0.00 |
| 0.1 mol% FeCl₃ | 50 | 3 | 0.0 | 96.23 | 0.00 | 0.86 | 0.04 | 1.65 |
| 0.1 mol% FeCl₃ | 60 | 3 | 0.0 | 95.37 | 0.00 | 1.83 | 0.04 | 0.74 |
| 0.1 mol% FeCl₃ | 70 | 3 | 0.0 | 93.48 | 0.06 | 4.20 | 0.05 | 1.27 |
| 0.1 mol% FeCl₃ | 80 | 3 | 0.0 | 91.54 | 0.00 | 5.97 | 0.22 | 1.38 |
| 0.05 mol% FeCl₃ | 60 | 3 | 0.0 | 91.67 | 0.00 | 5.85 | 0.30 | 0.13 |
| 0.01 mol% FeCl₃ | 60 | 3 | 0.0 | 80.20 | 0.08 | 16.92 | 0.76 | 0.00 |

Chloroprene (2-CP) was completely converted into higher chlorinated products in all experiments carried out. Benzene (BE) was almost completely reacted in most cases, but were also chlorinated to hexachlorobenzene (HCB) at high FeCl₃ concentrations (0.1 mol%). At low Fe concentrations, the chlorination of benzene resulted in the formation of chlorobenzene (CB) and dichlorobenzene. In all experiments, chlorination of dichloroethane (EDC) to trichloroethene (TCE) was also observed, possibly followed by the formation of still higher chlorinated products. The formation of TCE increased as expected as the temperature increased. But that the TCE formation increased already at low Fe concentrations and without using a catalyst was surprising. FeCl₃ was effective for the chlorination of benzene even at low concentrations (0.01 mol%). However, a high Fe concentration has a positive effect of suppressing the free-radical chlorination of EDC to TCE.

### Example 2: Chlorination using iron-aluminium(oxide) as catalyst

The catalytic tests were carried out according to the method defined under experimental procedures. The results of the screening are listed in table 2.

**Table 2: Chlorination using iron-aluminium(oxide) as catalyst**

| Catalyst [mg] | T [°C] | Time [h] | X_{CP} [%] | X_{B} [%] | EDC peak area [%] | BE peak area [%] | TCE peak area [%] | CB peak area [%] | HCB peak area [%] |
|---|---|---|---|---|---|---|---|---|---|
| 500 mg Alumnia (46105) | 70 | 1 | 100 | 72 | 97.55 | 0.30 | 0.66 | 0.66 | 0.00 |
| 500 mg FeₐAl_{b}O_{c} | 70 | 1 | 100 | >99 | 97.60 | <0.01 | 0.26 | 0.00 | 0.00 |
| 250 mg FeₐAl_{b}O_{c} | 70 | 1 | 100 | >99 | 98.12 | <0.01 | 0.12 | 0.37 | 0.00 |
| 250 mg FeₐAl_{b}O_{c} | 80 | 1 | 100 | 100 | 97.94 | 0.00 | 0.14 | 0.44 | 0.00 |

A commercial γ-alumina (Alfa Aesar, 46105) with a BET surface area of 380 m²/g, a mean pore diameter of 4 nm and a pore volume of 0.5 ml/g was tested for comparison. The sample was crashed and sieved. The fraction of granules <100 µm was used for the experiments without any additional pre-treatment. With 0.50 g alumina, the benzene conversion was 72 % at 70°C and after 1 h, while the chloroprene was fully converted.

The best catalyst was the iron-aluminium(oxide)-catalyst containing 10 wt.% Fe with a surface area of 183 m²/g. The catalyst has a mean pore diameter of 10 nm and a pore volume of about 0.6 ml/g. With 500 and 250 mg of the catalyst, benzene was completely converted, mainly to 1,2- and 1,4-dichlorobenzene. An extra experiment using the same catalyst was done at 80°C, and benzene as fully converted as it is shown in table 2. The conversion (X) of benzene (B) and chloroprene (CP) were calculated based on the peak area % from the GC analysis.

In conclusion, the iron-aluminium(oxide) as catalyst is highly selective and active, whilst commercial γ-alumina is less active and iron(III) chloride is less selective.

### Experimental procedures

### Catalyst preparation

The iron-aluminium(oxide)-catalyst was synthesized using a commercial water dispersible alumina (Disperal P2, Sasol). First a dispersion of alumina was prepared by stirring 80 g of water with 20 g of the alumina source in a 200 ml beaker at room temperature (at 22°C). After 1 h stirring, 10 g of Triton X100 was added and stirred for another 30 minutes. The magnet was removed and then a solution of 13.5 g of Fe(NO₃)3·9H₂O in 25 g of water was added to the dispersion and homogenized using a spatula.

The formed brown gel was left to dry in air until a weight loss of 50 %. The paste was homogenized and shaped using a syringe of 2 mm opening diameter. Then this was left for drying at room temperature for two days. Then, it was dried overnight at 80 °C. The calcination was done inside a tubular furnace with a heating rate of 1 K/min under the flow of air (150 ml/min) up to 300 °C, and then a portion of this catalyst was calcined again at 800 °C for 3 h (heating rate 10 K/min). Finally, 10 wt.% of iron supported on aluminium(oxide) was yielded.

### GC-MS Analysis

For GC analyses, the conditions used for separation are listed in table 3. Both GC (Agilent 6890) and GC-MS (Agilent 6890) devices were used for the analysis using similar columns. The temperature program of GC started at 50 °C for 6 min, and then the temperature was increased by 20 K/min up to 250 °C (holding time 7 min); the total run time was 23 min. For GC-MS, the temperature program started with 40°C for 5 min and then the temperature was raised with 20 K/min to 160 °C (no holding time), followed by a temperature ramp of 45 K/min to 250 °C (holding time 17 min); the total run time was 30 min. In both cases, the pure sample was injected without any dilution. 1 µl of the product solution was injected into the GC and 0.2 µl was used for the GC-MS analysis.

**Table 3: Conditions of GC and GC/MS used for analysis of products**

| **Method** | **Column name** | **Detector** | **Injector** | **Column temperature program** |
|---|---|---|---|---|
| GC | HPS (50 m x 0.32 mm x 0.52 µm) | FID (280 °C) | (250 °C) | 50 °C (6 min) (20 K/min) to 250 °C for (7 min) (20 K/min), total run time = 23 min |
| GC-MC | HPS (30 m x 0.25 mm x 0.25 µm) | FID (320 °C), MS (230 °C) | (260°C) | 40 °C (5 min) (20 K/min) to 160 °C (0 min) (45 K/min) to 250 °C (17 min), total run time = 30 min |

### Procedure testing iron(III) chloride

The reactions were realized as follows: at first, an autoclave was tested for leakage at 2 bar argon pressure. Afterwards, the autoclave was flushed several times with Cl₂ gas up to 1.8 bar at room temperature (at 22 °C). Whenever the pressure dropped to 0.5 bar, the inlet valve was opened for pressurizing the autoclave with fresh Cl₂ gas. This procedure was repeated until the pressure was constant (that means that the solution was saturated with dissolved chlorine). Subsequently, heating and stirring (700 rpm) was started until the reaction temperature, i.e. 50 °C. 60 °C, 70 °C or 80 °C, was reached. If the pressure inside the autoclave was higher than 2 bars due to the increase of temperature, the outlet valve was opened to release the excess pressure and to keep the pressure constant during the whole experiment close to 2 bars. As a result, there might be some loss of the reactants upon releasing the excess pressure. The experiment was left with the above conditions for 3 h. With this procedure, a high amount of ca. 30 mmol chlorine at 2 bar was applied for 30 g of EDC containing 2 mmol benzene. The amount of catalyst used was 0.01 mol% (= 100 mg Fe/liter substrate), 0.05 mol% (= 500 mg Fe/liter substrate) or 0.1 mol% (= 1000 mg Fe/liter substrate).

### Procedure testing iron-aluminium(oxide)

The procedure testing iron-aluminium(oxide) was performed as described for the iron(III) chloride. However, a low amount of Cl₂ gas of ca. 5 mmol was fed in order to achieve an incomplete conversion of benzene, to render the catalyst activity more comparable. The reaction temperature was fixed at 70°C. At first, the autoclave was checked for leakage at 2 bar argon pressure. Then it was purged two times with argon. The argon pressure inside the autoclave was fully released and heating to 70°C under stirring (700 rpm) started. When the temperature was reached, the inlet valve for Cl₂ gas was opened (one full round) for 10 sec. Initially, the internal pressure reached 1.5 bar while filling and then it decreased to 1-1.3 bar after closing the valve as Cl₂ gas was partially dissolved. The duration of an experiment was 1 h. The autoclave cooled down and then the pressure was released and a sample was taken for analysis on GC and/or GC/MS. The injection of the product samples was carried out instantly.

## Claims

1. A process of removing by-products from a vinyl chloride comprising process stream from a 1,2-dichloroethane thermal cracking process comprising the steps of:
(a) providing a starting material comprising 1,2-dichloroethane;
(b) thermally cracking 1,2-dichloroethane by heating the starting material to obtain a product mixture comprising vinyl chloride, hydrogen chloride, uncracked 1,2-dichloroethane and by-products;
(c) separating the hydrogen chloride and separating the vinyl chloride from the product mixture thereby obtaining a process stream comprising uncracked 1,2-dichloroethane and by-products;
(d) chlorinating the obtained process stream by adding liquid and/or gaseous chlorine to the process stream in the presence of a heterogeneous catalyst, thereby obtaining a process stream comprising the uncracked 1,2-dichloroethane, chlorinated by-products and, optionally, still by-products from step c);
(e) recovering the uncracked 1,2-dichloroethane from the process stream by separating the chlorinated by-products;
(f) optionally regenerating the catalyst through backflushing; and
wherein the heterogeneous catalyst is an iron-aluminium(oxide)-catalyst with a formula FeₐAl_{b}O_{c}.

2. The process of claim 1, wherein in step c) the vinyl chloride and the hydrogen chloride are separated through distillation.

3. The process of any of claim 1 or 2, wherein in step e) the uncracked 1,2-dichloroethane is recovered by separating the chlorinated by-products through distillation.

4. The process of any of claims 1 to 3, wherein in step d) the chlorinated by-products comprise high boiling chlorinated by-product, having a boiling temperature of at least 85 °C.

5. The process of any of claims 1 to 4, wherein the by-products comprise one or more hydrocarbons selected from the list comprising benzene, chloroprene and other hydrocarbons.

6. The process of any of claims 1 to 5, wherein the iron-aluminium(oxide)-catalyst is a catalytic fixed bed reactor.

7. The process of any of claims 1 to 6, wherein the starting material in step a) comprises 70 wt.% or more 1,2-dichloroethane, preferably 80 wt.% or more 1,2-dichloroethane, further preferably 90 wt.% or more 1,2-dichloroethane.

8. The process of any of claims 1 to 7, wherein in step b) 1,2-dichloroethane is thermally cracked, in an endothermic reaction, by a temperature ranging from 450 to 650 °C, preferably 450 to 600 °C, further preferably 450 to 550 °C.

9. The process of any of claims 1 to 8, wherein the cracking reaction in step b) is incomplete, having a conversion rate of 20 to 99 % based on total throughput of 1,2-dichloroethane, preferably 50 to 60 % based on total throughput of 1,2-dichloroethane.

10. The process of any of claims 1 to 9, wherein in step d) the temperature for carrying out the chlorination is 20 to 100 °C, preferably 40 to 90 °C, further preferably 60 to 80 °C.

11. The process of any of claims 1 to 10, wherein in step d) the pressure for carrying out the chlorination is 0.5 to 3 bar, preferably 0.5 to 2 bar.

12. The process of any of claims 1 to 11, wherein in step d) the amount of chlorine per kg EDC for carrying out the chlorination is 0.1 to 20 g, preferably 1.5 to 15 g, further preferably 2 to 4 g.

13. The process of any of claims 1 to 12, wherein in step d) the chlorine is provided as a gaseous chlorine.

14. Use of a heterogeneous catalyst in a process of removing by-products from a vinyl chloride comprising process stream from a 1,2-dichloroethane thermal cracking process **characterized in that** the catalyst is an iron-aluminium(oxide)-catalyst with a formula FeₐAl_{b}O_{c}.

15. The use of claim 14, wherein the by-products are chlorinated by adding liquid and/or gaseous chlorine to the process stream in the presence of the iron-aluminium(oxide)-catalyst.

## Patentansprüche

1. Verfahren zur Entfernung von Nebenprodukten aus einem Vinylchlorid umfassenden Prozessstrom aus einem Prozess für das thermische Cracken von 1,2-Dichlorethan, umfassend die Schritte:
(a) Bereitstellen eines Ausgangsstoffs, umfassend 1,2-Dichlorethan;
(b) thermisches Cracken von 1,2-Dichlorethan durch Erwärmen des Ausgangsmaterials, um ein Produktgemisch zu erhalten, umfassend Vinylchlorid, Chlorwasserstoff, ungecracktes 1,2-Dichlorethan und Nebenprodukte;
(c) Abtrennen des Chlorwasserstoffs und Abtrennen des Vinylchlorids aus dem Produktgemisch, wodurch ein Prozessstrom erhalten wird, welcher ungecracktes 1,2-Dichlorethan und Nebenprodukte umfasst;
(d) Chlorieren des erhaltenen Prozessstroms durch Zugabe von flüssigem und/oder gasförmigem Chlor zu dem Prozessstrom in Gegenwart eines heterogenen Katalysators, um dadurch einen Prozessstrom zu erhalten, umfassend das ungecrackte 1,2-Dichlorethan, chlorierte Nebenprodukte und, optional, noch Nebenprodukte aus Schritt c);
(e) Rückgewinnung des ungecrackten 1,2-Dichlorethan aus dem Prozessstrom durch Abtrennen der chlorierten Nebenprodukte;
(f) optional das Regenerieren des Katalysators durch Rückspülung; und
wobei der heterogene Katalysator ein Eisen-Aluminium(oxid)-Katalysator ist, mit der Formel FeₐAl_{b}O_{c}.

2. Verfahren nach Anspruch 1, wobei in Schritt c) das Vinylchlorid und der Chlorwasserstoff abgetrennt werden durch Destillation.

3. Verfahren nach einem der Ansprüche 1 oder 2, wobei in Schritt e) das ungecrackte 1,2-Dichlorethan zurückgewonnen wird durch Abtrennen der chlorierten Nebenprodukte mittels Destillation.

4. Verfahren nach einem der Ansprüche 1 bis 3, wobei in Schritt d) die chlorierten Nebenprodukte hochsiedende chlorierte Nebenprodukte umfassen, aufweisend eine Siedetemperatur von wenigstens 85 °C.

5. Verfahren nach einem der Ansprüche 1 bis 4, wobei die Nebenprodukte einen oder mehr Kohlenwasserstoffe umfassen, ausgewählt aus der Liste umfassend Benzol, Chloropren und andere Kohlenwasserstoffe.

6. Verfahren nach einem der Ansprüche 1 bis 5, wobei der Eisen-Aluminium(oxid)-Katalysator ein katalytischer Festbettreaktor ist.

7. Verfahren nach einem der Ansprüche 1 bis 6, wobei das Ausgangsmaterial in Schritt a) 70 Gew.-% oder mehr an 1,2-Dichlorethan, vorzugsweise 80 Gew.-% oder mehr an 1,2-Dichlorethan umfasst, weiterhin bevorzugt 90 Gew.-% oder mehr an 1,2-Dichlorethan.

8. Verfahren nach einem der Ansprüche 1 bis 7, wobei in Schritt b) 1,2-Dichlorethan thermisch gecrackt wird in einer endothermen Reaktion mittels einer Temperatur im Bereich von 450 bis 650 °C, vorzugsweise 450 bis 600 °C, weiterhin bevorzugt 450 bis 550 °C.

9. Verfahren nach einem der Ansprüche 1 bis 8, wobei die Crack-Reaktion in Schritt b) unvollständig ist, mit einer Umwandlungsrate von 20 bis 99 %, basierend auf dem Gesamtdurchsatz an 1,2-Dichlorethan, vorzugsweise 50 bis 60 %, basierend auf dem Gesamtdurchsatz an 1,2-Dichlorethan.

10. Verfahren nach einem der Ansprüche 1 bis 9, wobei in Schritt d) die Temperatur zur Durchführung der Chlorierung 20 bis 100 °C beträgt, vorzugsweise 40 bis 90 °C, weiterhin bevorzugt 60 bis 80 °C.

11. Verfahren nach einem der Ansprüche 1 bis 10, wobei in Schritt d) der Druck zur Durchführung der Chlorierung 0,5 bis 3 bar beträgt, vorzugsweise 0,5 bis 2 bar.

12. Verfahren nach einem der Ansprüche 1 bis 11, wobei in Schritt d) die Menge an Chlor pro kg EDC zur Durchführung der Chlorierung 0,1 bis 20 g beträgt, vorzugsweise 1,5 bis 15 g, weiterhin bevorzugt 2 bis 4 g.

13. Verfahren nach einem der Ansprüche 1 bis 12, wobei in Schritt d) das Chlor bereitgestellt wird als gasförmiges Chlor.

14. Verwendung eines heterogenen Katalysators in einem Verfahren zur Entfernung von Nebenprodukten aus einem Vinylchlorid umfassenden Prozessstrom aus einem thermischen 1,2-Dichlorethan-Cracking-Prozess, **dadurch gekennzeichnet, dass** der Katalysator ein Eisen-Aluminium(oxid)-Katalysator ist, mit der Formel FeₐAl_{b}O_{c}.

15. Verwendung nach Anspruch 14, wobei die Nebenprodukte chloriert werden durch Zugabe von flüssigem und/oder gasförmigem Chlor zum Prozessstrom in Gegenwart des Eisen-Aluminium(oxid)-Katalysators.

## Revendications

1. Procédé d'élimination des sous-produits d'un chlorure de vinyle comprenant un courant de procédé provenant d'un procédé de craquage thermique de 1,2-dichloroéthane, comprenant les étapes consistant à :
(a) fournir une matière première comprenant du 1,2-dichloroéthane ;
(b) craquer thermiquement le 1,2-dichloroéthane en chauffant la matière première pour obtenir un mélange de produits comprenant du chlorure de vinyle, du chlorure d'hydrogène, du 1,2-dichloroéthane non craqué et des sous-produits ;
(c) séparer le chlorure d'hydrogène et le chlorure de vinyle du mélange de produits et obtenir ainsi un courant de procédé comprenant du 1,2-dichloroéthane non craqué et des sous-produits ;
(d) chlorer le courant de procédé obtenu en ajoutant du chlore liquide et/ou gazeux au courant de procédé en présence d'un catalyseur hétérogène, ce qui permet d'obtenir un courant de procédé comprenant le 1,2-dichloroéthane non craqué, des sous-produits chlorés et, éventuellement, des sous-produits encore présents à l'étape c) ;
(e) récupérer le 1,2-dichloroéthane non craqué du courant de procédé en séparant les sous-produits chlorés ;
(f) régénérer éventuellement le catalyseur par rétro-rinçage ; et
dans lequel le catalyseur hétérogène est un catalyseur à base d'oxyde de fer et d'aluminium de formule FeₐAl_{b}Oc_{c}.

2. Procédé de la revendication 1, dans lequel, à l'étape c), le chlorure de vinyle et le chlorure d'hydrogène sont séparés par distillation.

3. Procédé de l'une quelconque des revendications 1 ou 2, dans lequel, à l'étape e), le 1,2- dichloroéthane non craqué est récupéré en séparant les sous-produits chlorés par distillation.

4. Procédé de l'une quelconque des revendications 1 à 3, dans lequel, à l'étape d), les sous-produits chlorés comprennent un sous-produit chloré à point d'ébullition élevé, dont la température d'ébullition est d'au moins 85 °C.

5. Procédé de l'une quelconque des revendications 1 à 4, dans lequel les sous-produits comprennent un ou plusieurs hydrocarbures choisis dans la liste comprenant le benzène, le chloroprène et d'autres hydrocarbures.

6. Procédé de l'une quelconque des revendications 1 à 5, dans lequel le catalyseur à base d'oxyde de fer et d'aluminium est un réacteur catalytique à lit fixe.

7. Procédé de l'une quelconque des revendications 1 à 6, dans lequel la matière première de l'étape a) comprend 70 % en poids ou plus de 1,2-dichloroéthane, de préférence 80 % en poids ou plus de 1,2-dichloroéthane, de préférence encore 90 % en poids ou plus de 1,2-dichloroéthane.

8. Procédé de l'une quelconque des revendications 1 à 7, dans lequel, à l'étape b), le 1,2-dichloroéthane est craqué thermiquement, dans une réaction endothermique, à une température comprise entre 450 et 650 °C, de préférence entre 450 et 600 °C, de préférence encore entre 450 et 550 °C.

9. Procédé de l'une quelconque des revendications 1 à 8, dans lequel la réaction de craquage à l'étape b) est incomplète, avec un taux de conversion de 20 à 99 % par rapport au débit total de 1,2-dichloroéthane, de préférence de 50 à 60 % par rapport au débit total de 1,2-dichloroéthane.

10. Procédé de l'une quelconque des revendications 1 à 9, dans lequel, à l'étape d), la température de réalisation de la chloration est comprise entre 20 et 100 °C, de préférence entre 40 et 90 °C, de préférence encore entre 60 et 80 °C.

11. Procédé de l'une quelconque des revendications 1 à 10, dans lequel, à l'étape d), la pression pour effectuer la chloration est de 0,5 à 3 bars, de préférence de 0,5 à 2 bars.

12. Procédé de l'une quelconque des revendications 1 à 11, dans lequel, à l'étape d), la quantité de chlore par kg d'EDC pour effectuer la chloration est comprise entre 0,1 et 20 g, de préférence entre 1,5 et 15 g, de préférence encore entre 2 et 4 g.

13. Procédé de l'une quelconque des revendications 1 à 12, dans lequel, à l'étape d), le chlore est fourni sous forme de chlore gazeux.

14. Utilisation d'un catalyseur hétérogène dans un procédé d'élimination des sous-produits d'un courant de procédé comprenant du chlorure de vinyle provenant d'un procédé de craquage thermique du 1,2-dichloroéthane, **caractérisé en ce que** le catalyseur est un catalyseur à base d'oxyde de fer et d'aluminium dont la formule est FeₐAl_{b}O_{c}.

15. Utilisation de la revendication 14, dans laquelle les sous-produits sont chlorés par l'ajout de chlore liquide et/ou gazeux au courant de procédé en présence du catalyseur à base d'oxyde de fer et d'aluminium.
